**Europäisches Patentamt**

**European Patent Office**

**Office européen des brevets**

(11) Veröffentlichungsnummer : **0 362 773 B1**

# (12) EUROPÄISCHE PATENTSCHRIFT

(45) Veröffentlichungstag der Patentschrift :
**31.08.94 Patentblatt 94/35**

(21) Anmeldenummer : **89118250.3**

(22) Anmeldetag : **02.10.89**

(51) Int. Cl.⁵ : **G01N 33/68**, // C07K7/08

(54) Verfahren zur Bestimmung von Osteocalcin in humanem Serum oder Plasma.

(30) Priorität : **05.10.88 DE 3833936**

(43) Veröffentlichungstag der Anmeldung :
**11.04.90 Patentblatt 90/15**

(45) Bekanntmachung des Hinweises auf die
Patenterteilung :
**31.08.94 Patentblatt 94/35**

(84) Benannte Vertragsstaaten :
**AT BE CH DE ES FR GB GR IT LI LU NL SE**

(56) Entgegenhaltungen :
**EP-A- 0 095 873**
**US-A- 4 410 506**
**CLINICAL ORTHOPAEDICS AND RELATED**
**RESEARCH, Band 226, Jänner 1988, Philadel-**
**phia, PA (US) ; J.B. LIAN et al., Seiten 267-291**

(73) Patentinhaber : **Henning Berlin Anlagen GmbH**
**Komturstrasse 58-62**
**D-12099 Berlin (DE)**

(72) Erfinder : **Bergmann, Andreas Eberhard, Dr.**
**Neudecker Weg 119**
**D-1000 Berlin 42 (DE)**

(74) Vertreter : **Andrae, Steffen, Dr.**
**Patentanwälte**
**Andrae, Flach, Haug, Kneissl**
**Balanstrasse 55**
**D-81541 München (DE)**

## Beschreibung

Die vorliegende Erfindung betrifft ein Verfahren zur Bestimmung von Osteocalcin in humanem Serum oder Plasma. Das Verfahren ist ein Immunoassay, bei dem eine Probe des das zu bestimmende hOsteocalcin enthaltenden Serums oder Plasmas zusammen mit einer definierten Menge eines nachfolgend näher definierten Oligopeptidtracers, der eine mit geeigneten physikalischen Nachweismethoden nachweisbare Markierungsgruppe enthält, mit einem Antikörper inkubiert wird, der einen spezifischen Binder für das Osteocalcin und den Oligopeptidtracer darstellt, wonach der Anteil des an den Antikörper gebundenen Oligopeptidtracers anhand seiner Markierung ermittelt und daraus rechnerisch die Menge des in dem Serum oder Plasma enthaltenen hOsteocalcins bestimmt wird.

Humanes Osteocalcin, das auch als Vitamin K-abhängiges Knochenprotein oder γ-Carboxyglutaminsäure enthaltendes Protein (im englischsprachigen Schrifttum bone Gla protein (BGP)) bezeichnet wird, ist ein spezifischer Peptidbestandteil der Knochenmatrix. Das Peptid besteht aus 49 Aminosäuren und weist die folgende Aminosäuresequenz auf:

Tyr-Leu-Tyr-Gln-Trp-Leu-Gly-Ala-Pro-Val-Pro-Tyr-Pro-Asp-Pro-Leu-
Glu-Pro-Arg-Arg-Gla-Val-Cys-Gla-Leu-Asn-Pro-Asp-Cys-Asp-Glu-Leu-
Ala-Asp-Arg-Ile-Gly-Phe-Gln-Glu-Ala-Tyr-Arg-Arg-Phe-Tyr-Gly-Pro-
Val$^{49}$

Das Peptid wird von den Osteoblasten synthetisiert, und ein geringer Teil des gebildeten Osteocalcins wird in das Blut freigesetzt. Über die Bestimmung der Konzentration dieses Osteocalcins im Blut sind bestimmte wertvolle Rückschlüsse auf den Stoffwechsel des Knochens möglich, wobei zur ergänzenden Information, auf die Arbeit von Lian, J.B. und Gundberg, C.M. in: Clinical Orthopaedics and Related Research, 226, Seite 267 bis 291 (1988) verwiesen wird. Über die Bestimmung der Osteocalcinkonzentration in humanem Serum oder Plasma können danach möglicherweise hilfreiche Informationen für die Diagnose und Therapie von Knochenstoffwechselstörungen erhalten werden.

Zur Messung der Osteocalcinkonzentration wurden verschiedene immundiagnostische Verfahren, insbesondere Radioimmunoassays, entwickelt. Die vorliegende Erfindung betrifft eine für seine praktische Anwendbarkeit wichtige erfinderische Weiterentwicklung eines immundiagnostischen Nachweisverfahrens, das auf der Lehre der US-A-4 438 208 beruht. Gemäß dem genannten US-Patent werden verschiedene Oligopeptide, die Bruchstücke des intakten hOsteocalcins darstellen, insbesondere das C-terminale Tridecapeptid von hOsteocalcin (hOsteocalcin 37-49),zur Erzeugung von gegen diesen Peptidabschnitt des humanen Osteocalcins gerichteten Antikörpern verwendet, die dann für einen Radioimmunoassay zur Bestimmung des intakten hOsteocalcins 1-49 eingesetzt werden. Bei dem nach dem bekannten Konkurrenzprinzip arbeitenden Immunoassay wird als Tracer ein Oligopeptidtracer verwendet, der ein markiertes Osteocalcin 37-49 oder vorzugsweise als Osteocalcin-Analogtracer 38-49 ein Oligopeptid mit der folgenden Struktur darstellt:

Tyr$^{38}$-Gln-Glu-Ala-Phe-Arg-Arg-Phe-Phe-Gly-Pro-Val$^{49}$

Die Bindung dieses Analogtracers an den Anti-Osteocalcin 37-49 Antikörper vermindert sich mit steigender Konzentration an Osteocalcin 1-49 in der untersuchten Probe. Die in der Probe (Inkubationsansatz) vorhandene Menge an Osteocalcin 1-49 kann daher auf übliche Weise unter Verwendung einer Osteocalcin 37-49-Standardkurve quantifiziert werden. Gemäß US-PS 4 438 208 wird angenommen, daß ein solcher Test für die Bestimmung des humanen Osteocalcins in z. B. humanem Serum geeignet sei.

Gegenüber einem Immunoassay, der unter Verwendung von Antikörpern gegen das vollständige Osteocalcin bzw. unter Verwendung von Osteocalcin 1-49-Tracern arbeitet, hat das Verfahren gemäß US-PS 4 438 208 zahlreiche wichtige Vorteile:

Der Antikörper ist gegen eine Aminosäuresequenz gerichtet, die einer bestimmten Region des humanen Osteocalcins entspricht, und zwar dessen C-terminalem Teil. In diesem Teil befindet sich keine Calcium-Bindungsstelle, woraus folgt, daß die Wechselwirkung Osteocalcin 1-49/Antikörper von der Kalziumkonzentration unabhängig ist.

Ferner kann der oben erwähnte Analogtracer 38-49 verwendet werden. Dieser ist ein Analogpeptid, das nur eine Tyrosineinheit enthält. Damit ist der Tracer nach einer Radiojodierung (z. B. mittels der bekannten Chloramin T-Methode) leicht in reiner Form zu gewinnen (z. B. über HPLC). Osteocalcin 1-49 enthält 5 Tyrosineinheiten,und auch das Oligopeptid Osteocalcin 37-49 noch 2 Tyrosineinheiten. Werden diese Peptide radiojodiert, entstehen in der Regel mehrere verschieden markierte Produkte, die für eine Verwendung als Tracer aufgetrennt werden müssen, was Probleme verursacht.

Bei dem Versuch einer Bestimmung des Osteocalcins in humanem Serum (oder Plasma) wurde jedoch festgestellt, daß die gemessenen Werte in Abhängigkeit von den Inkubationsbedingungen unterschiedlich ausfielen und daß häufig zu hohe Osteocalcin-Werte in der zu testenden Probe vorgetäuscht wurden.

Es ist Aufgabe der vorliegenden Erfindung, ein Verfahren zur Bestimmung von hOsteocalcin, bei dem eine das zu bestimmende Osteocalcin enthaltende Probe einer biologischen Flüssigkeit zusammen mit einer definierten Menge eines Oligopeptidtracers, insbesondere eines Osteocalcinanalogtracers 38-49, der eine mit geeigneten Nachweismethoden nachweisbare Markierungsgruppe enthält, mit einem Antikörper inkubiert wird, der einen spezifischen Binder für das Osteocalcin und den Oligopeptidtracer darstellt, wonach der Anteil des an den Antikörper gebundenen Oligopeptidtracers anhand seiner Markierung ermittelt und daraus rechnerisch die Menge des in dem Serum oder Plasma enthaltenen Osteocalcins bestimmt wird, so auszugestalten, daß eine zuverlässige und reproduzierbare Bestimmung des hOsteocalcins in einem humanen Serum oder Plasma ermöglicht wird.

Diese Aufgabe wird bei einem Verfahren gemäß Oberbegriff von Patentanspruch 1 durch die im Kennzeichen wiedergegebene Maßnahme gelöst.

Bevorzugte vorteilhafte Ausgestaltungen des Grundverfahrens gemäß Patentanspruch 1 sind in den Unteransprüchen wiedergegeben.

Grundlage des erfindungsgemäßen Verfahrens ist die Erkenntnis des Erfinders,daß die fehlerhaften Meßergebnisse und die zu hohen ermittelten Osteocalcin-Werte in Proben von humanem Serum oder Plasma darauf beruhen, daß in diesen Proteasen vorhanden sind, welche während der Inkubation von Tracer, Antikörper und Probe den Analogtracer schnell fragmentieren. Die dabei gebildeten Fragmente des Analogtracers werden von dem Antikörper nicht mehr gebunden, so daß aufgrund der verminderten Mengen des an den Antikörper gebundenen Tracers falsche, nämlich zu hohe Osteocalcin-Werte in der zu testenden Probe vorgetäuscht werden. Gleichzeitig wurde auch noch festgestellt, daß auch das Oligopeptid Osteocalcin 37-49 in Proben von humanem Serum oder Plasma ähnlich schnell wie der Analogtracer abgebaut wird. Diese Untersuchungsergebnisse erklärten die beobachteten fehlerhaften Meßergebnisse und erlaubten den Schluß, daß Osteocalcin mittels des Verfahrens gemäß US-A-4 438 208 nicht direkt in humanem serum oder Plasma zuverlässig bestimmt werden kann.

Nachdem jedoch die Erkenntnis gewonnen war, daß das Problem der proteolytische Abbau der verwendeten Oligopeptidtracer ist, konnten Lösungsstrategien zur Lösung dieses Problems entwickelt werden, die dann letztlich zur Schaffung des erfindungsgemäßen Verfahrens führten. Wenn der proteolytische Abbau des Oligopeptidtracers das zu lösende Problem darstellt, schien es sinnvoll zu versuchen, zumindest für die Dauer der Inkubation von Tracer, Antikörper und Serum/Plasma die Aktivität der den Tracer abbauenden Proteasen so weit zu reduzieren, daß der Tracer stabil bleibt.

Zur Verminderung der Proteaseaktivitäten schienen verschiedene Maßnahmen möglich: So konnte daran gedacht werden, die Proteasen von dem zu messenden Osteocalcin zu separieren, z. B. durch Extraktion des Osteocalcins aus der Serums- bzw. Plasmaprobe. Die Extraktion von Patientenseren/plasmen ist jedoch eine aufwendige Methode, die zeit- und kostenaufwendig ist und daher für die Praxis wenig geeignet erscheint.

Als weitere Maßnahme konnte an eine thermische Denaturierung und damit Inaktivierung der Proteasen gedacht werden. Eine Hitzebehandlung von Seren/Plasmen ist jedoch nur bis zu einer Temperatur von 58°C möglich, da bei höheren Temperaturen Bestandteile im Serum/Plasma ausflocken, was wiederum negative Folgen für die Genauigkeit eines Osteocalcin-Radioimmunoassays haben kann. Es zeigte sich dabei jedoch, daß Seren, die 1 h bei 58°C behandelt wurden, im Hinblick auf die Aktivität des beobachteten Osteocalcin 38-49 (Analogtracer)-Abbaus keine wesentlichen Veränderungen zeigten. Die für eine Wärmebehandlung von Seren mögliche Maximaltemperatur von 58°C reicht somit nicht aus, die in den Proben vorhandenen Proteasen zu inaktivieren.

Als weitere mögliche Maßnahme konnte die Inhibierung der Proteasen durch den Zusatz geeigneter Hemmstoff in Erwägung gezogen werden. Da nicht bekannt war, welche Proteasen in dem humanen Serum und Plasma vorhanden sind bzw. im Hinblick auf die Fragmentierung von Osteocalcin 38-49 (Analogtracer) wirksam sind, war nicht voraussagbar, ob eine derartige Maßnahme der Hemmung der Proteasen mit geeigneten Hemmstoffen möglich ist und wenn ja, welche Hemmstoffe dann zur Lösung der gestellten Aufgabe wirksam sein würden.

Nach umfangreichen Untersuchungen, die in Tabelle 1 zusammengefaßt sind, zeigte sich, daß es nicht möglich war, den Abbau des unveränderten Osteocalcins 38-49 (Analogtracers) durch Zugabe eines einzelnen Protease-Hemmstoffs zu unterdrücken. Als daraus geschlossen wurde, daß humanes Serum oder Plasma mehr als eine den Oligopeptidtracer abbauende Protease enthält, und als daraufhin Mischungen von Protease-Hemmstoffen untersucht wurden, zeichnete sich eine Lösung der der Erfindung zugrundeliegenden Aufgabe ab.

Es wurde nämlich festgestellt, daß eine Mischung von Leupeptin und Amastatin, die beide allein den Abbau des Oligopeptids nicht ausreichend unterdrückten, den Abbau des Oligopeptidtracers verhindert.

Auch dieses Ergebnis kann der nachfolgenden Tabelle 1 entnommen werden.

Tabelle 1: Einfluss verschiedener Protease-Inhibitoren auf die Hydrolyse von $^{125}$I-Osteocalcin 38-49 (Analogtracer)

| Hemmstoff | Konzentration | %der Hydrolyse-geschwindigkeit der Kontrolle |
|---|---|---|
| Kontrolle | / | 100 |
| Ethylendiamin-tetraacetat | 10mM | 100 |
| Diisopropyl-fluorophosphat | 1mM | 100 |
| Phenylmethyl-sulfonylfluorid | 2mM | 100 |
| p-Chloromercury-phenylsulfonsäure | 1mM | 100 |
| N-Ethylmaleimid | 2mM | 100 |
| Bestatin | 1mM | 100 |
| Amastatin | 100µM | 88 |
| Pepstatin | 100µM | 100 |
| Elastatinal | 100µM | 100 |
| Leupeptin | 1mM | 100 |

| Phosphoramidon | 1mM | 100 |
| Benzamidin | 1mM | 100 |
| Trasylol | $1,6 \cdot 10^6$ Einheiten/ml | 100 |
| Heparin | 5mg/ml | 100 |
| Trypsin-Inhibitor (Soybean) | 0.1mg/ml | 100 |
| Antithrombin III | 0.1 Einheiten/ml | 100 |
| Hitzebehandlung (1h/ 58°C) | / | 100 |
| Leupeptin | 1mM + | |
| Amastatin | 100µM | <2 |

Die Proteolyse des mit $^{125}$I markierten Osteocalcins 38-49 (Analogtracers) wurde dabei wie nachfolgend in den Beispielen näher beschrieben verfolgt.

Die überraschende Wirksamkeit der Kombination von Leupeptin und Amastatin im Hinblick auf die Fragmentierung von Osteocalcin 38-49 (Analogpeptid) und auch von - wie sich in anschließenden Untersuchungen herausstellte - Osteocalcin 38-49 in den gleichen biologischen Flüssigkeiten gestattete dann aufgrund der bekannten hemmenden Wirkungen von Leupeptin bzw. Amastatin den Schluß, daß die Fragmentierung des Oligopeptidtracer sowohl durch eine (oder verschiedene) Endopeptidase(n) (Leupeptin ist ein Endopeptidase-Hemmstoff) als auch eine (oder verschiedene) Aminopeptidase(n) (Amastatin ist ein Aminopeptidase-Hemmstoff) bewirkt wird.

Da von Aminopeptidasen bekannt ist, daß diese nur Peptide spalten, wenn die Aminogruppe der N-terminalen Aminosäure frei vorliegt, konnte eine weitere Variante der Lösung der erfindungsgemäßen Aufgabe aufgefunden werden: Durch Blockierung der freien Aminogruppe (z. B. durch Acetylierung) läßt sich das Oligopeptid gegen einen Abbau durch die Aminopeptidasen schützen. Wird somit ein Oligopeptid mit einer geschützten Aminogruppe am N-Terminus eingesetzt und gleichzeitig ein Hemmstoff gegen Endopeptidasen (z. B. Leupeptin) eingesetzt,läßt sich der Abbau des in geschützter Form eingesetzten Oligopeptidtracers ebenfalls verhindern.

Während das geschützte Oligopeptid im Serum/Plasma weiterhin schnell fragmentiert wird, genügt in diesem Falle der Zusatz von Leupeptin allein, um diesen Abbau vollständig zu unterdrücken. Die nachfolgende Tabelle 2 faßt diese Ergebnisse zusammen. Danach wird ($^{125}$I)-N-Acetyl-Osteocalcin 38-49 (Analogpeptid) im Serum/Plasma schnell fragmentiert. Der Zusatz von Leupeptin in der erforderlichen Mindestkonzentration unterdrückt jedoch diesen Abbau.

Tabelle 2 :  Effekt von Leupeptin auf den Abbau von $^{125}$I-N-Acetyl-Osteocalcin 38-49 (Analogpeptid)

| Hemmstoff | Konzentration | %der Hydrolyse-geschwindigkeit der Kontrolle |
| --- | --- | --- |
| Kontrolle | / | 100 |
| Leupeptin | 50µM | 0 |

Der Abbau wurde wie in den Beispielen näher beschrieben verfolgt.

Die erfindungsgemäße Aufgabe wird somit damit gelöst, daß die Bestimmung von Osteocalcin in einer Probe von humanem Serum oder Plasma in Gegenwart von einem oder mehreren den proteolytischen Abbau des verwendeten Oligopeptidtracers verhindernden Proteolysehemmstoffen erfolgt.

Wird ein ungeschützter Oligopeptidtracer, beispielsweise der bevorzugte Osteocalcin 38-49 (Analogpeptid) Oligopeptidtracer verwendet, müssen geeignete Proteolysehemmstoffe gegen die im humanen Serum oder Plasma vorhandene Endopeptidase sowie die Aminopeptidase verwendet werden. Diese Proteolysehemmstoffe müssen dabei in der jeweils für eine wirksame Hemmung erforderlichen Mindestkonzentration im Inkubationsansatz verwendet werden.

Im Falle des bevorzugten Endopeptidase-Hemmstoffs Leupeptin beträgt diese Mindestkonzentration 50 µM, im Falle des bevorzugten Aminopeptidase-Hemmstoffs Amastatin beträgt diese Mindestkonzentration 25 µM.

Die genannten Proteolyse-Hemmstoffe Leupeptin und Amastatin sind bekannte Substanzen und auch im Handel erhältlich. Amastatin wird beschrieben z. B. in dem Artikel "Inhibitors of metalloproteases" von James C. Powers and J. Wade Harper in "Proteinase Inhibitors", Herausgeber Barrett and Salvesen, 1986 Elsevier Science Publishers BV (Biomedical Division). Leupeptin ist ebenfalls ein bekannter Protease-Inhibitor und wird beispielsweise unter dem Stichwort "Inhibitorpeptides" in der "Concise Encyclopedia of Biochemistry", de Gruyter, 1983, erwähnt.

Das erfindungsgemäße Verfahren wird vorzugsweise als Radioimmunoassay (RIA) durchgeführt, wobei der Oligopeptidtracer mit $^{125}$I radioaktiv markiert ist. Anstelle der genannten radioaktiven Markierung kann jedoch auch irgendeine andere auf dem Gebiet der Immundiagnostik verwendete Markierung zum Einsatz kommen, beispielsweise andere Radionuklide, Enzyme, fluoreszierende Reste, chemilumineszierende Reste, magnetische Teilchen, stabile freie Radikale usw.

Der im erfindungsgemäßen Verfahren eingesetzte Antikörper kann sowohl ein polyklonaler als auch ein monoklonaler Antikörper sein.

Dieser Antikörper kann in gelöster oder dispergierter Form vorliegen, ist jedoch vorzugsweise durch Bindung an eine geeignete feste Phase immobilisiert, was die Trennung von gebundener und freier Phase erleichtert. Beim Arbeiten in homogener Phase kommt vorzugsweise die bekannte Doppelantikörper-Technik zur Anwendung.

Die durch das erfindungsgemäße Verfahren geschaffene Möglichkeit der direkten Bestimmung von Osteocalcin in humanem Serum oder Plasma ist eine wichtige Voraussetzung für die routinemäßige Osteocalcinbestimmung in der klinischen Praxis.

Nachfolgend werden die zur Schaffung der vorliegenden Erfindung führenden Bestimmungen noch genauer erläutert.

## $^{125}$I-Osteocalcin 38-49 (Analogtracer)

Der Analogtracer wurde durch Radiojodierung nach der bekannten Chloramin-T-Methode unter Verwendung des als Handelsprodukt (Bachem) erhältlichen Osteocalcins 38-49 hergestellt.

## $^{125}$I-N-Acetyl-Osteocalcin 38-49 Analogtracer

Durch Umsetzung von Osteocalcin 38-49 (Analogpeptid) mit Essigsäureanhydrid wurde zuerst das N-terminal geschützte N-Acetyl-Osteocalcin 38-49 Analogpeptid hergestellt. Dieses wurde anschließend nach einer Reinigung auf dem Wege der Umkehrphasen-Hochdruck-Flüssigkeitschromatographie (RPLC) mittels der bekannten Chloramin-T-Methode radiojodiert. Das radiojodierte Produkt wurde wiederum über RPLC gereinigt.

## Untersuchung der Hemmung der proteolytischen Fragmentierung von Osteocalcin-Oligopeptiden und -Analogpeptiden

$^{125}$I-Osteocalcin 38-49 Analogpeptid, N-Acetyl-$^{125}$I-Osteocalcin 38-49 Analogpeptid und $^{125}$I-Osteocalcin 37-49 wurden jeweils in Mengen von 2 x 10$^6$ cpm in 5 µl Wasser mit 40 µl Puffer (25 mM Natriumphosphat, 3 mM EDTA, 0,1% NaN$_3$, pH 7,8) vermischt. Für die Kontrollmessung wurde reiner Puffer verwendet, für die Untersuchung der verschiedenen Proteolysehemmstoffe wurden diese in Konzentrationen, die zu den in Tabelle 1 bzw. 2 angegebenen Konzentrationen im Inkubationsansatz führten, mit dem Puffer zugegeben. Anschließend wurden 5 µl humanes Serum oder Plasma zugesetzt, und es folgte eine 10minütige Inkubation bei 37°C.

Danach wurde die Reaktion durch Zusatz von 100 µl HCl (0,2 N) gestoppt. Die erhaltene Reaktionsmischung wurde auf den Abbau des Peptids hin untersucht. Die Analyse des jeweiligen eingesetzten Peptids und seiner Abbauprodukte erfolgte über HPLC unter Verwendung einer µBondapak C$_{18}$-Säule (0,4 x 30 cm).

Die Elution der Substanzen erfolgte unter Verwendung eines Gradienten, der aus einem Lösungsmittel A (LM A) aus Acetonitril : Wasser : Trifluoressigsäure im Volumenverhältnis 5 : 95 : 0,1 und einem Lösungsmittel B (LM B) aus Acetonitril : Wasser : Trifluoressigsäure im Volumenverhältnis 90 : 10 : 0,1 wie folgt erzeugt wurde:

In 3 min linear von 100/0 (V/V)(LM A/ LM B) nach 85/15 LM A/ LM B und anschließend in 14 min linear auf 50/50 LM A/ LM B. Die Durchflußgeschwindigkeit betrug 1,5 ml pro min. Die Radioaktivität des Eluats wurde kontinuierlich mittels eines Radioaktivitätsmonitors (Raytest) verfolgt. Der Abbau der radioaktiven Peptide (in Prozent Umsatz) wurde aus dem resultierenden Abbaumuster unter Verwendung eines Computerprogrammes (Raytest) ermittelt.

Es zeigte sich, daß die in die Abbauversuche eingesetzten Peptide sich bei ihrer Untersuchung mittels HPLC von einer Vielzahl ihrer Fragmente abtrennen ließen, so daß der Abbau der Peptide leicht untersucht werden kann. Ohne Zusatz von Proteolyse-Hemmstoffen oder bei Zusatz ungeeigneter Proteolyse-Hemmstoffe wird bei 37°C unter den angegebenen Bedingungen nach einer 10minütigen Inkubation mit Serum oder Plasma ein etwa 80%iger Abbau des eingesetzten Tracers beobachtet.

Dieser Abbau wird im Falle der ungeschützten Peptide zu über 99 % unterdrückt, wenn den Inkubationsansätzen gleichzeitig Leupeptin und Amastatin in Konzentrationen oberhalb einer für den jeweiligen Hemmstoff charakteristischen Mindestkonzentration zugesetzt werden. Diese Mindestkonzentrationen des Hemmstoffs müssen im Zeitpunkt der Inkubation des Peptids (Tracers) mit dem Serum oder Plasma für Leupeptin mindestens 50 µM und für Amastatin mindestens 25 µM betragen.

Die Hemmwirkung einer Mischung aus Leupeptin und Amastatin wurde danach auch noch unter anderen Inkubationsbedingungen, die den üblichen Bedingungen für die Durchführung eines Osteocalcin-Radioimmunoassays entsprachen, untersucht. Auch bei einer derartigen 20stündigen Inkubation bei 4°C wird in Anwesenheit der Mischung der genannten Proteolyse-Hemmstoffe kein Abbau des Tracers beobachtet.

Tabelle 1 faßt die Ergebnisse für die Inkubation bei 37°C zusammen. Die eingesetzten Proteolysehemmstoffe wurden im Handel von den nachfolgend in Klammern angegebenen Bezugsquellen bezogen: Amastatin (NovaBiochem); Leupeptin (Bachem); Bestatin (Bachem); Elastatinal (Sigma); Pepstatin (NovaBiochem); Phosphoramidon (NovaBiochem); Benzamidin (Sigma); Trasylol (Bayer); Trypsin-Inhibitor from Soybean (Sigma); Antithrombin III (Sigma).

Abbauversuche des N-Acetyl-$^{125}$I-Osteocalcin 38-49 Analogpeptids

Bei einer 10minütigen Inkubation bei 37°C unter den obigen Bedingungen mit humanem Serum oder Plasma werden etwa 70 % des eingesetzten acetylierten Analogpeptids abgebaut.

Wird den Inkubationsansätzen Leupeptin (Mindestkonzentration 50 µM)zugesetzt, wird dieser Abbau vollständig unterdrückt.

Wie im Falle der ungeschützten Peptide, verhindert die Anwesenheit von Leupeptin auch unter den Bedingungen der Durchführung eines Osteocalcin-Radioimmuno-Assays vollständig den Abbau des geschützten Analogtracers.

Diese Ergebnisse sind in Tabelle 2 zusammengefaßt.

Radioimmunoassays

Es wurde festgestellt, daß die Bindungskurven für N-Acetyl-$^{125}$I-Osteocalcin 38-49 Analogpeptid (für den geschützten Analogtracer) bei seiner Verwendung in einem Osteocalcin-Radioimmunoassay denen des ungeschützen Osteocalcin 38-49 Analogpeptids sehr ähnlich sind, so daß auch der acetylierte Analogtracer in einem derartigen Radioimmunoassay als Oligopeptidtracer verwendet werden kann.

Es wurde festgestellt, daß der Zusatz von Amastatin und Leupeptin zwar den proteolytischen Tracerabbau zuverlässig verhinderte, ansonsten den Osteocalcin-Radioimmunoassay nicht nennenswert beeinflußt.

Alle Radioimmunoassay-Untersuchungen wurden unter Verwendung des "OSCAtest-Osteocalcin (37-49) human (Bone Gla Protein, BGP)" der Firma Henning Berlin GmbH nach den für diesen Radioimmunoassay angegebenen Vorschriften durchgeführt.

**Patentansprüche**

1. Verfahren zur Bestimmung von Osteocalcin in humanem Serum oder Plasma, bei dem eine Probe des das zu bestimmende Osteocalcin enthaltenden Serums oder Plasmas zusammen mit einer definierten Menge eines Oligopeptidtracers, der eine mit geeigneten physikalischen Nachweismethoden nachweisbare Markierungsgruppe enthält, mit einem Antikörper inkubiert wird, der einen spezifischen Binder für das Osteocalcin und den Oligopeptidtracer darstellt, wonach der Anteil des an den Antikörper gebundenen Oligopeptidtracers anhand seiner Markierung ermittelt und daraus rechnerisch die Menge des in dem Serum oder Plasma enthaltenen Osteocalcins bestimmt wird,

dadurch gekennzeichnet, daß

der Probe des humanen Serums oder Plasmas vor oder gemeinsam mit der Zugabe des Oligopeptidtracers ein oder mehrere den proteolytischen Abbau des verwendeten Oligopeptidtracers verhindernde(r) Proteolysehemmstoff(e) zugesetzt wird (werden).

2. Verfahren nach Anspruch 1, dadurch gekennzeichnet, daß der Oligopeptidtracer ein an sich bekanntes markiertes Oligopeptid aus einer Sequenz von 10 bis 16 Aminosäuren ist, das die C-terminalen Aminosäuren von humanem Osteocalcin umfaßt, von denen bis zu drei Aminosäuren durch andere Aminosäuren ausgetauscht sein können, mit der Maßgabe, daß höchstens ein derartiger Aminosäureaustausch kein Austausch Phe/Tyr oder Glu/Gla ist, und daß als Proteolysehemmstoffe ein Endopeptidase-Hemmstoff und ein Aminopeptidase-Hemmstoff in der jeweils erforderlichen Mindestkonzentration in Mischung miteinander verwendet werden.

3. Verfahren nach Anspruch 2, dadurch gekennzeichnet, daß eine solche Mischung aus Amastatin und Leupeptin verwendet wird, daß in dem die Probe, den Oligopeptidtracer und den Antikörper enthaltenden Ansatz eine Mindestkonzentration von Amastatin von 25 µM und eine Mindestkonzentration von Leupeptin von 50 µM vorliegen.

4. Verfahren nach Anspruch 2 oder 3, dadurch gekennzeichnet, daß als Oligopeptidtracer ein im Tyr-Rest mit radioaktivem Jod markiertes Oligopeptid der folgenden Aminosäuresequenz verwendet wird (hOsteocalcin-Analogfragment 38-49):

$$\underline{\text{Tyr}}^{38}\text{-Gln-Glu-Ala-Phe-Arg-Arg-Phe-Phe-Gly-Pro-Val}^{49}$$

5. Verfahren nach Anspruch 1, dadurch gekennzeichnet, daß der Oligopeptidtracer ein an sich bekanntes markiertes Oligopeptid aus einer Sequenz von 10 bis 16 Aminosäuren ist, das die C-terminalen Aminosäuren von Osteocalcin umfaßt, von denen bis zu drei Aminosäuren durch andere Aminosäuren ausgetauscht sein können, mit der Maßgabe, daß höchstens ein derartiger Aminosäureaustausch kein Austausch Phe/Tyr oder Glu/Gla ist, und dessen N-terminale Aminosäure in geschützter Form vorliegt, und daß als Proteolysehemmstoff ein geeigneter Endopeptidase-Hemmstoff in der erforderlichen Mindestkonzentration verwendet wird.

6. Verfahren nach Anspruch 5, dadurch gekennzeichnet, daß als Oligopeptidtracer ein im Tyr-Rest mit radioaktivem Jod markiertes Oligopeptid der nachfolgenden Aminosäuresequenz verwendet wird, dessen N-terminale Aminosäure geschützt ist (N-terminal geschütztes hOsteocalcin-Analogfragment 38-49):

$$\underline{\text{Tyr}}^{38}\text{-Gln-Glu-Ala-Phe-Arg-Arg-Phe-Phe-Gly-Pro-Val}^{49}$$

7. Verfahren nach Anspruch 5 oder 6, dadurch gekennzeichnet, daß ein durch Acetylierung der N-terminalen Aminosäure geschütztes Oligopeptid verwendet wird.

8. Verfahren nach einem der Ansprüche 5 bis 7, dadurch gekennzeichnet, daß als Endopeptidase-Hemmstoff Leupeptin in einer solchen Konzentration verwendet wird, daß es in dem die Probe, den Oligopeptidtracer und den Antikörper enthaltenden Ansatz in einer Mindestkonzentration von 50 µM Leupeptin vorliegt.

## Claims

1. Method for the determination of osteocalcin in human serum or plasma, in which a sample of the serum or plasma containing the osteocalcin to be determined is incubated, together with a defined amount of an oligo-peptide tracer which contains a labelling group detectable by suitable physical detection methods, with an antibody which represents a specific binder for the osteocalcin and the oligopeptide tracer, after which the proportion of oligopeptide tracer bound to the antibody is measured on the basis of its labelling and, from this, the amount of osteocalcin contained in the serum or plasma is determined by calculation, characterized in that one or more proteolysis inhibitor(s) preventing the proteolytic breakdown of the oligopeptide tracer which is used is (are) added to the sample of the human serum or plasma before or together with the addition of the oligopeptide tracer.

2. Method according to Claim 1, characterized in that the oligopeptide tracer is a labelled oligopeptide which is known per se and is composed of a sequence of 10 to 16 amino acids which contains the C-terminal amino acids of human osteocalcin, up to three amino acids of which can be replaced by other amino acids, with the proviso that a maximum of one amino acid replacement of this type is not a Phe/Tyr or Glu/Gla replacement, and that used as proteolysis inhibitors are an endopeptidase inhibitor and an aminopeptidase inhibitor mixed together in the minimum concentration required in each case.

3. Method according to Claim 2, characterized by using a mixture of amastatin and leupeptin such that a minimum concentration of amastatin of 25 $\mu$M and a minimum concentration of leupeptin of 50 $\mu$M are present in the mixture containing the sample, the oligopeptide tracer and the antibodies used.

4. Method according to Claim 2 or 3, characterized in that an oligopeptide of the following amino acid sequence (h-osteocalcin fragment 38-49 analogue):

$$\underline{Tyr}^{38}-Gln-Glu-Ala-Phe-Arg-Arg-Phe-Phe-Gly-Pro-Val^{49}$$

which is labelled with radioactive iodine in the Tyr residue is used as oligopeptide tracer.

5. Method according to Claim 1, characterized in that the oligopeptide tracer is a labelled oligopeptide which is known per se and is composed of a sequence of 10 to 16 amino acids which contains the C-terminal amino acids of human osteocalcin, up to three amino acids of which can be replaced by other amino acids, with the proviso that a maximum of one amino acid replacement of this type is not a Phe/Tyr or Glu/Gla replacement, and whose N-terminal amino acid is present in protected form, and that a suitable endopeptidase inhibitor in the minimum concentration required is used as proteolysis inhibitor.

6. Method according to Claim 5, characterized in that an oligopeptide of the following amino acid sequence, whose N-terminal amino acid is protected (N-terminally protected h-osteocalcin fragment 38-49 analogue):

$$\underline{Tyr}^{38}-Gln-Glu-Ala-Phe-Arg-Arg-Phe-Phe-Gly-Pro-Val^{49}$$

and which is labelled with radioactive iodine in the Tyr residue is used as oligopeptide tracer.

7. Method according to Claim 5 or 6, characterized in that an oligopeptide protected by acetylation of the N-terminal amino acid is used.

8. Method according to one of Claims 5 to 7, characterized in that leupeptin is used as endopeptidase inhibitor in a concentration such that it is present in the mixture containing the sample, the oligopeptide tracer and the antibody in a minimum concentration of 50 $\mu$M leupeptin.


## Revendications

1. Procédé de détermination de l'ostéocalcine dans le sérum ou le plasma humain, dans lequel un échantillon du sérum ou du plasma contenant l'ostéocalcine à déterminer est mis à incuber avec un anticorps, ensemble avec une quantité définie d'un traceur oligopeptidique qui contient un radical de marquage décelable par des procédés physiques de détection appropriés, l'anticorps constituant un ligand spécifique de l'ostéocalcine et du traceur oligopeptidique, suite à quoi la fraction du traceur oligopeptidique lié à l'anticorps est déterminée à l'aide de son marquage, la quantité d'ostéocalcine contenue dans le sérum ou le plasma en étant déduite par calcul,
caractérisé en ce que
l'on ajoute à l'échantillon de sérum ou de plasma humain, avant ou en même temps que l'addition du traceur oligopeptidique, un ou plusieurs inhibiteurs de la protéolyse, pour empêcher la décomposition protéolytique du traceur oligopeptidique utilisé.

2. Procédé selon la revendication 1, caractérisé en ce que le traceur oligopeptidique est un oligopeptide marqué, connu en soi, constitué d'une séquence de 10 à 16 acides aminés, qui comprend les acides aminés C-terminaux de l'ostéocalcine humaine, dont deux à trois acides aminés peuvent être remplacés par d'autres acides aminés dans la mesure où, autant que possible, un tel échange d'acides aminés n'est pas un échange Phe/Tyr ni Glu/Gla,
et en ce que l'on utilise comme inhibiteurs de la protéolyse un inhibiteur d'endopeptidase et un inhibiteur d'aminopeptidase mélangés l'un à l'autre, dans la concentration minimale chaque fois nécessaire.

3. Procédé selon la revendication 2, caractérisé en ce que l'on utilise un mélange d'anastatine et de leupeptine, tel que dans l'ensemble contenant l'échantillon, le traceur oligopeptidique et l'anticorps, il règne une concentration minimale en anastatine de 25 $\mu$M et une concentration minimale en leupeptine de 50 $\mu$M.

4. Procédé selon la revendication 2 ou 3, caractérisé en ce que l'on utilise comme traceur oligopeptidique un oligopeptide marqué à l'iode radioactif sur le résidu Tyr, présentant la séquence d'acides aminés suivante (fragment 38-49 analogue à l'h-ostéocalcine):

$$\underline{\mathrm{Tyr}}^{38}\mathrm{-Gln-Glu-Ala-Phe-Arg-Arg-Phe-Phe-Gly-Pro-Val}^{49}.$$

5. Procédé selon la revendication 1, caractérisé en ce que le traceur oligopeptidique est un oligopeptide marqué, connu en soi, constitué d'une séquence de 10 à 16 acides aminés, qui comprend les acides aminés C-terminaux de l'ostéocalcine, dont deux à trois acides aminés peuvent être remplacés par d'autres acides aminés dans la mesure où, autant que possible, un tel échange d'acides aminés n'est pas un échange Phe/Tyr ni Glu/Gla, et dont les acides aminés N-terminaux sont présents sous forme protégée,
et en ce que l'on utilise comme inhibiteur de la protéolyse un inhibiteur d'endopeptidase approprié, dans la concentration minimale nécessaire.

6. Procédé selon la revendication 5, caractérisé en ce que l'on utilise comme traceur oligopeptidique un oligopeptide marqué à l'iode radioactif sur le résidu Tyr, présentant la séquence d'acides aminés suivante, dont les acides aminés N-terminaux sont protégés (fragment 38-49 analogue à l'h-ostéocalcine, protégé en positions N-terminales):

$$\underline{\mathrm{Tyr}}^{38}\mathrm{-Gln-Glu-Ala-Phe-Arg-Arg-Phe-Phe-Gly-Pro-Val}^{49}.$$

7. Procédé selon la revendication 5 ou 6, caractérisé en ce que l'on utilise un oligopeptide protégé par acétylation des acides aminés N-terminaux.

8. Procédé selon l'une des revendications 5 à 7, caractérisé en ce que l'on utilise comme inhibiteur d'endopeptidase, de la leupeptine à une concentration telle que dans l'ensemble contenant l'échantillon, le traceur oligopeptidique et l'anticorps, il règne une concentration minimale en leupeptine de 50 $\mu$M.